# EUROPEAN PATENT SPECIFICATION

(11) **EP 2 589 334 B1**
(45) Date of publication and mention of the grant of the patent: **29.07.2015**
(21) Application number: 11800726.9
(22) Date of filing: 23.06.2011
(51) Int. Cl.: A61B 5/05

(54) **BODY COMPOSITION MEASUREMENT DEVICE**
VORRICHTUNG ZUM MESSEN EINER KÖRPERZUSAMMENSETZUNG
DISPOSITIF DE MESURE DE COMPOSITION DE CORPS

(30) Priority: 25.10.2010 JP 2010238711; 01.07.2010 JP 2010151119
(43) Date of publication of application: 08.05.2013
(73) Proprietor: Panasonic Corporation, Kadoma-shi Osaka 571-8501 (JP)
(72) Inventor: FUKUDA, Hiroaki, Osaka 540-6207 (JP); TAKAHASHI, Tatsuya, Osaka 540-6207 (JP); FUKUSHIMA, Shogo, Osaka 540-6207 (JP); OCHI, Kazuhiro, Osaka 540-6207 (JP)
(74) Representative: Grünecker Patent- und Rechtsanwälte PartG mbB
(86) International application number: PCT/JP2011/064478
(87) International publication number: WO 2012/002261

(56) References cited:
- WO-A1-2007/083622
- JP-A- 2001 178 697
- JP-A- 2001 252 256
- JP-A- 2001 252 257
- JP-A- 2002 369 806
- JP-A- 2007 014 578
- JP-A- 2009 225 854
- JP-A- 2009 254 667
- US-A1- 2004 077 969
- US-A1- 2005 222 516
- US-A1- 2006 025 701
- US-A1- 2008 306 401
- US-B1- 6 978 170

## Description

The present invention relates to a body composition measurement device.

Patent document 1 describes a body fat measurement device that performs a regression analysis with a single voltage measurement value, which is measured by a voltage electrode pair, and a measured subject parameter to estimate a total body fat amount of a measured subject.

### (Prior art document)

Patent Document 1: Japanese Laid-Open Patent Publication No. 2002-369806
Patent document 2: US 6,978,170 B1 disclosing a body fat measuring apparatus measuring the thickness of subcutaneous fat at a plurality of points along a circumference of a subject Patent document 3: US 2005/0222516 A1 disclosing electrode position correction arm portions supported on a sliding mechanism 2 (Problems that are to be solved by the invention)

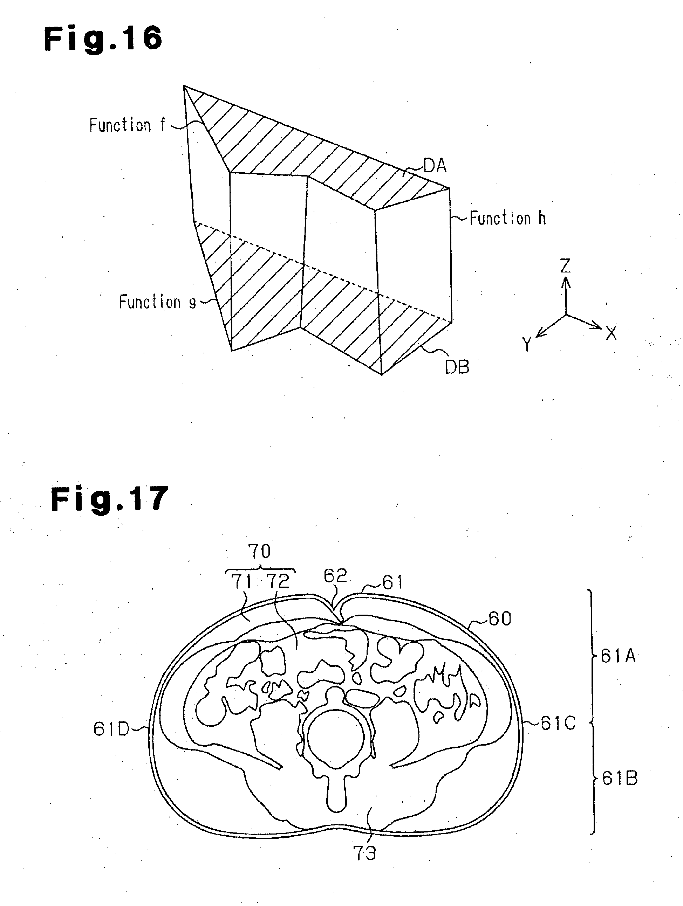

Referring to Fig. 17, body fat 70 includes visceral fat 72 and subcutaneous fat 71. The body fat 70 and muscle 73 are arranged unevenly in a cross-section perpendicular to the trunk axis. However, the inventors of the present invention have noticed that the body fat amount, which is estimated by performing the regression analysis on a single voltage measurement value, does not reflect the distribution of the body fat 70, and the body fat amount cannot be measured with satisfactory accuracy.

Accordingly, it is an object of the present invention to provide a body composition measurement device that can measure a body fat amount reflecting the distribution of body fat.

### (Means for solving the problem)

A body composition measurement device according to one aspect of the present invention includes a device according to claim 1.

Preferably, the voltage electrode pairs are configured to measure voltages for a measured subject in the vicinity of a frontal plane and at an anterior belly.

In one example, the control unit calculates, as the body fat amount, an area of body fat in a measured cross-section of a measured subject.

In one example, the control unit estimates a distribution of body fat in a measured cross-section of a measured subject.

In one example, the control unit measures visceral fat amount as the body fat amount.

In one example, the control unit measures subcutaneous fat amount as the body fat amount.

In one example, the control unit performs a first integration using measurement values of a plurality of measurement points on a first measured cross-section of a measured subject and a second integration using measurement values of a plurality of measurement points on a second measured cross-section, and the control unit calculates a body fat volume as the body fat amount based on a calculation result of the first integration and a calculation result of the second integration.

Preferably, the current electrode pair includes a first current electrode and a second current electrode arranged on opposite sides of a measured subject, and the voltage electrode pairs are arranged between the first current electrode and the second current electrode.

In one example, the current electrode pair includes a first current electrode, which is arranged on a frontal plane of a right body half of a measured subject, and a second current electrode, which is arranged on a frontal plane of a left body half.

The body composition measurement device of one example further includes a subcutaneous fat measurement means for measuring subcutaneous fat. The control unit calculates the visceral fat amount as the body fat amount that reflects the subcutaneous fat amount.

Preferably, a distance is variable between two voltage electrodes of at least one of the voltage electrode pairs, and the control unit reflects the distance in the integration.

Preferably, the body composition measurement device further includes a distance measurement mechanism that measures the distance between the current electrode pair and two adjacent ones of electrodes forming the voltage electrode pairs.

In one example, the distance measurement mechanism includes a distance sensor that measures the distance between the two adjacent ones of electrodes.

In one example, the body composition measurement device further includes a first connection portion, which connects a first voltage electrode and a second voltage electrode, and a second connection portion, which connects the first voltage electrode and a third voltage electrode. The distance measurement mechanism includes an angle sensor that detects an angle between the first connection portion and the second connection portion.

In one example, the control unit estimates an abdominal circumference based on the distance between the two adjacent ones of the electrodes measured by the distance measurement mechanism.

In one example, the control unit stores the measurement values and positions of the measurement points in association with the corresponding measurement values and calculates the body fat amount based on the positions of the measurement points and the measurement values corresponding to the positions of the measurement points.

A body composition measurement device of one example includes a current electrode pair configured to apply current, a plurality of voltage electrode pairs configured to measure voltages at a plurality of measurement points, and a control unit connected to the current electrode pair and the voltage electrode pairs. A plurality of electrodes forming the current electrode pair and the voltage electrode pairs are arranged in series and can be arranged on an arc forming part of a profile of a measured cross-section of the measured subject. The control unit performs an integration based on measurement values measured by the voltage electrode pairs and positions of the measurement points to calculate a body fat amount.

### (Effect of the invention)

The present invention provides a body composition measurement device that can measure the body fat amount in correspondence with the distribution of body fat.
Fig. 1 is a block diagram of a body composition measurement device according to a first embodiment of the present invention.
Fig. 2 is a front view of a body fat measurement device in the first embodiment.
Figs. 3A and 3B are enlarged partial views illustrating the body fat measurement device of Fig. 2.
Figs. 4A and 4B are enlarged partial views illustrating the body fat measurement device of Fig. 2.
Figs. 5A and 5B are front views illustrating the body composition measurement device of Fig. 1.
Figs. 6A to 6C are front views illustrating electrodes drawn out from the body fat measurement device of Fig. 2.
Fig. 7A is a schematic diagram schematically illustrating the positions of the body composition measurement device and a measured subject, and Fig. 7B is a cross-sectional view illustrating the locations of the electrodes of the body fat measurement device.
Fig. 8 is a flowchart of a body fat measurement process.
Fig. 9 is a body fat distribution graph generated by the body fat measurement device.
Fig. 10 is an enlarged partial view illustrating a body composition measurement device according to a second embodiment of the present invention;
Fig. 11 is a schematic diagram schematically illustrating the positional relationship of the electrodes of a body fat measurement device and a measured subject in a body composition measurement device according to a fourth embodiment of the present invention.
Fig. 12 is a schematic diagram schematically illustrating the positional relationship of the electrodes and a measured subject in a further embodiment of the present invention.
Fig. 13 is a schematic diagram schematically illustrating the positional relationship of the electrodes and the measured subject in a modified example of the first embodiment of the present invention.
Fig. 14 is a schematic diagram schematically illustrating the positional relationship of the electrodes and the measured subject in a modified example of the first embodiment of the present invention.
Fig. 15 is a schematic diagram schematically illustrating the positional relationship of the electrodes and the measured subject in a modified example of the first embodiment of the present invention.
Fig. 16 is a body fat distribution graph generated by a body fat measurement device in a modified example of the first embodiment of the present invention.
Fig. 17 is a schematic diagram schematically illustrating one example of the body fat distribution for a human abdomen serving as a measured subject of the body fat measurement device in each embodiment.

### First Embodiment

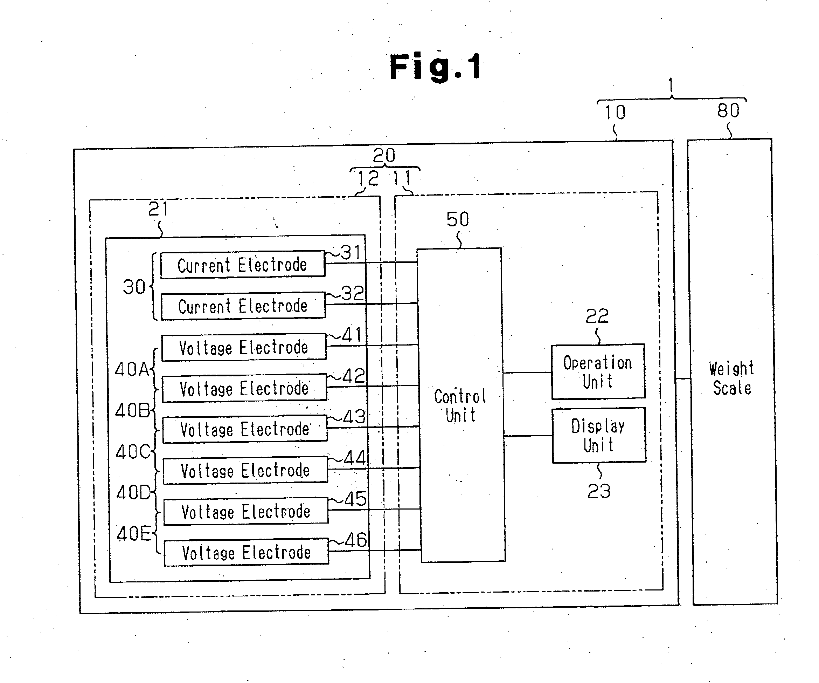

A first embodiment of the present invention will now be described with reference to Figs. 1 to 9.

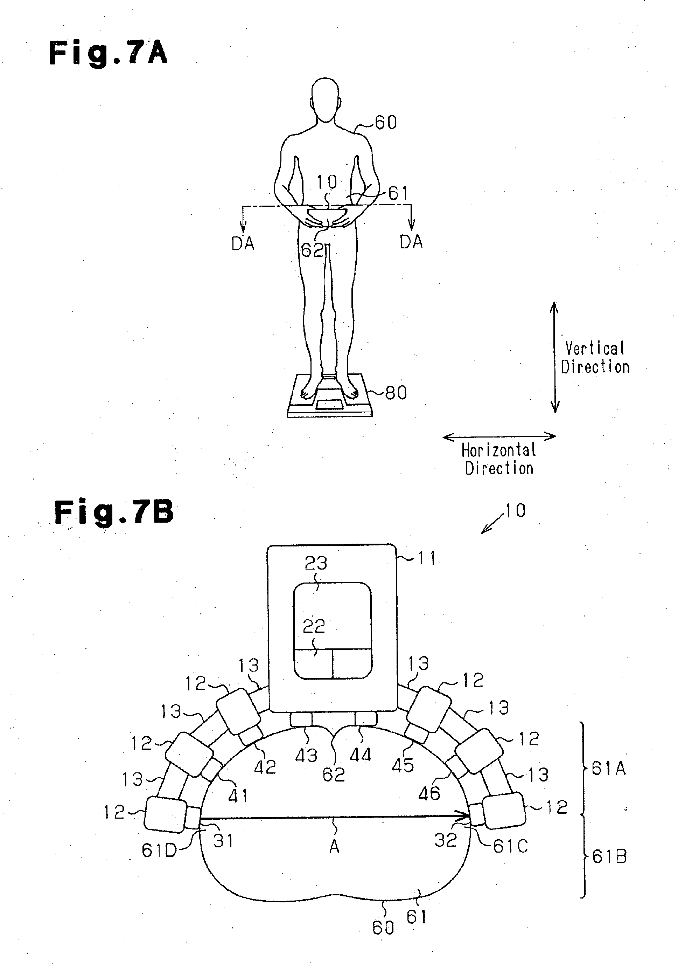

As illustrated in Fig. 1, a body composition measurement device 1 includes a body fat measurement device 10 and a weight scale 80, which can be connected to the body fat measurement device 10. Fig. 7A illustrates the body composition measurement device 1 used by a measured subject 60 (also referred to as a user). The body composition measurement device 1 may be operated by the measured subject 60 or a person other than the measured subject. In the description hereafter, the measured subject 60 and the person other than the measured subject may be referred to as the measuring person.

The body fat measurement device 10 includes a measurement portion 20 that measures body fat 70. The measurement portion 20 includes a detection unit 21, which applies current to the measured subject 60 and detects voltage, an operation unit 22, which is used to input the information required to measure the body fat 70, a display unit 23, which shows various information to the measuring person, and a control unit 50, which performs calculations using measurement results provided from the detection unit 21.

The detection unit 21 includes a first current electrode 31 and second current electrode 32, which apply current to the measured subject 60, and a first voltage electrode 41, second voltage electrode 42, third voltage electrode 43, fourth voltage electrode 44, fifth voltage electrode 45, and sixth voltage electrode 46, which measure the voltage of the measured subject 60.

The first current electrode 31 and second current electrode 32 form a current electrode pair 30. The first voltage electrode 41 and second voltage electrode 42 form a voltage electrode pair 40A. The third voltage electrode 43 and fourth voltage electrode 44 form a voltage electrode pair 40C. The fourth voltage electrode 44 and the fifth voltage electrode 45 form a voltage electrode pair 40D. The fifth voltage electrode 45 and sixth voltage electrode 46 form a voltage electrode pair 40E. The electrodes 31, 32, and 41 to 46 are each connected by a signal transmission line to the control unit 50.

When the measuring person operates the operation unit 22 to instruct the measurement of body fat 70 to be started, the control unit 50 starts the application of current with the current electrode pair 30. The control unit 50 calculates the body fat amount based on the voltage values measured by voltage electrodes 41 to 46, the input value input with the operation unit 22, and the weight measured by the weight scale 80. The calculation result mainly reflects the amount of visceral fat 72 located at a deep location in the body. The calculation result may include the area and distribution of the body fat 70 in the measured cross-section. The control unit 50 uses the calculation result to generate and display on the display unit 23 a graph based on the area and distribution of the body fat.

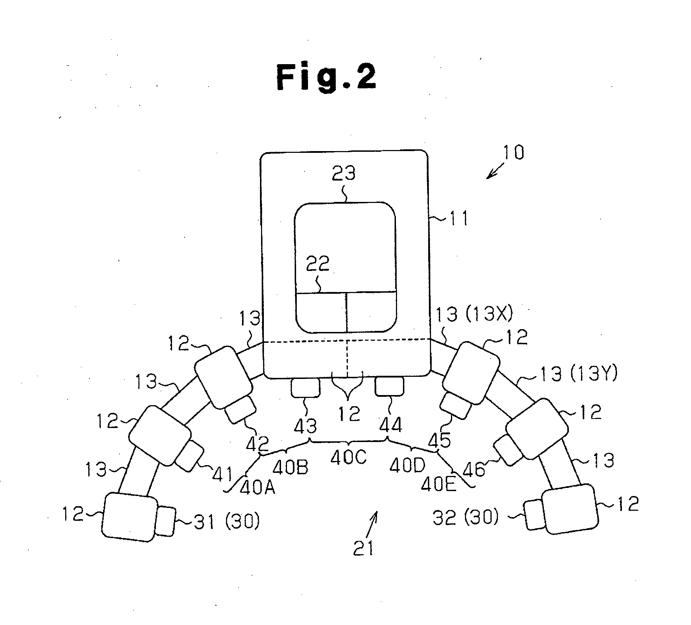

The structure of the body fat measurement device 10 will now be described with reference to Fig. 2.

The body fat measurement device 10 includes a main body 11 and a plurality of electrode seats 12. In the illustrated example, the eight electrodes 31, 32, and 41 to 46 are respectively connected to the eight electrode seats 12. The electrode material for the electrodes 31, 32, and 41 to 46 may be a stainless steel alloy or a metal-plated resin. The main body 11 can accommodate a power supply and the control unit 50, which are used by the body fat measurement device 10. The operation unit 22 and the display unit 23 may be exposed from the main body 11.

The electrode seats 12 are arranged in series. Some of the electrode seats 12 may be formed integrally with the main body 11. In the illustrated example,, the electrode seats 12 are connected so that the electrodes are arranged in the order of the first current electrode 31, the first voltage electrode 41, the second voltage electrode 42, the third voltage electrode 43, the fourth voltage electrode 44, the fifth voltage electrode 45, the sixth voltage electrode 46, and the second current electrode 32. The first and second current electrodes 31 and 32 are arranged at the two ends of the series-layout of the electrode seats 12. The third voltage electrode 43 and the fourth voltage electrode 44 are arranged in part of the main body 11 that serves as the electrode seats 12. A connection portion 13 connects two adjacent ones of the electrode seats 12. The structure of the connection portion 13 will now be described with reference to Figs. 2 to 4.

The variable length structure of the connection portion 13 will now be described. The connection portions 13 all have the same structure. Thus, as a representative, the connection portion 13 that connects the electrode seat 12 of the fifth voltage electrode 45 and the electrode seat 12 of the sixth voltage electrode 46 will be described.

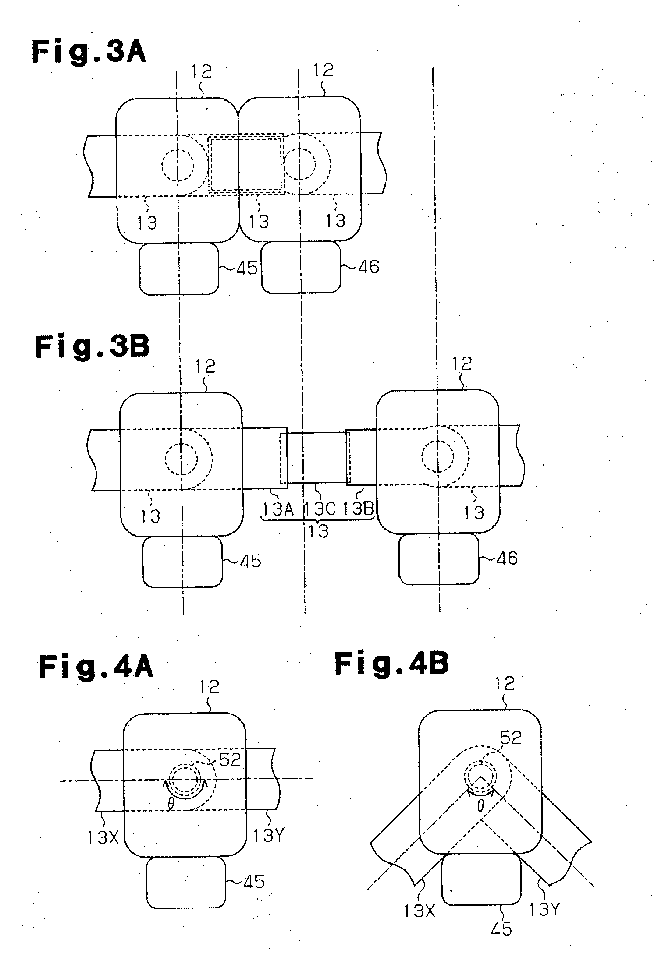

As illustrated in Figs. 3A and 3B, the connection portion 13 includes an outer pipe 13A, which is connected to the electrode seat 12 of the fifth voltage electrode 45, an inner pipe 13B, which is connected to the electrode seat 12 of the sixth voltage electrode 46, and an intermediate pipe 13C, which connects the outer pipe 13A and the inner pipe 13B. The inner pipe 13B and the intermediate pipe 13C are moved in the axial direction relative to the outer pipe 13A to lengthen and shorten the connection portion 13.

Fig. 3A illustrates a state in which the length of the connection portion 13 is minimum (minimum extension amount (zero)). Fig. 3B illustrates a state in which the length of the connection portion 13 is maximum (maximum extension amount). When the extension amount of the connection portion 13 is minimum, the electrode seat 12 of the fifth voltage electrode 45 comes into contact with the electrode seat 12 of the sixth voltage electrode 46. When the extension amount of the connection portion 13 is maximum, the electrode seat 12 of the fifth voltage electrode 45 is most separated from the electrode seat 12 of the sixth voltage electrode 46.

In the illustrated example, the connection portion 13 has a triplex structure formed by the outer pipe 13A, inner pipe 13B, and intermediate pipe 13C. However, the number of pipes and the lengths of the pipes can be varied in accordance with the desired maximum extension amount of the connection portion 13. For example, the connection portion 13 may be a dual structure, a quadruplex structure, or greater.

The connection portion 13 has a rotational structure that will now be described.

As illustrated in Figs. 2 and 4, a connection portion 13X and connection portion 13Y are connected to the same electrode seat 12 by a rotational hinge structure. The connection portion 13Y is rotated relative to the connection portion 13X to vary the angle θ between the longitudinal axis of the connection portion 13X and the longitudinal axis of the connection portion 13Y. The angle θ may be referred to as the bending angle of the electrode seat 12.

Fig. 4A illustrates a state in which the angle θ is maximum. The maximum value of the angle θ can be determined so that the connection portion 13X and the connection portion 13Y are arranged straight. Fig. 4B illustrates a state in which the angle θ is minimum. The minimum value of the angle θ can be determined so that the connection portion 13X is perpendicular to the connection portion 13Y.

It is preferred that the maximum extension amount of the connection portion 13 and the minimum value of the angle θ be determined based on the abdominal circumference of the typical measured subject 60. In this manner, the distance and angle between the electrode seats 12 can be varied in accordance with the measured subject 60.

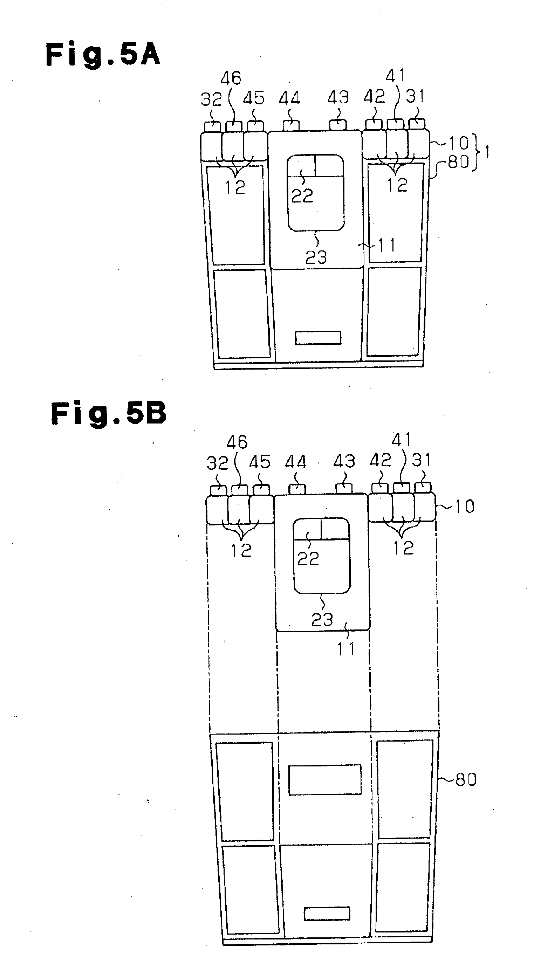

Referring to Figs. 5 to 7, the procedures for measuring the body fat with the body composition measurement device 1 will now be described. Fig. 7B illustrates a cross-section DA that extends through the umbilicus 62 of the abdomen 61 of the measured subject 60 and is perpendicular to the trunk axis of the measured subject 60 who is standing (refer to Fig. 7A). In this specification, the term "frontal plane" refers to a plane that is perpendicular to the cross-sectional DA of the measured subject 60, parallel to the trunk axis, and parallel to the sideward direction from the measured subject 60. The front side of the frontal plane is referred to as the anterior belly 61A, and the rear side of the frontal plane is referred to as the back 61B. In the body surfaces that intersect the frontal plane, the right half of the body is referred to as the right flank 61C, and the left half of the body is referred to as the left flank 61 D (refer to Fig. 7B).

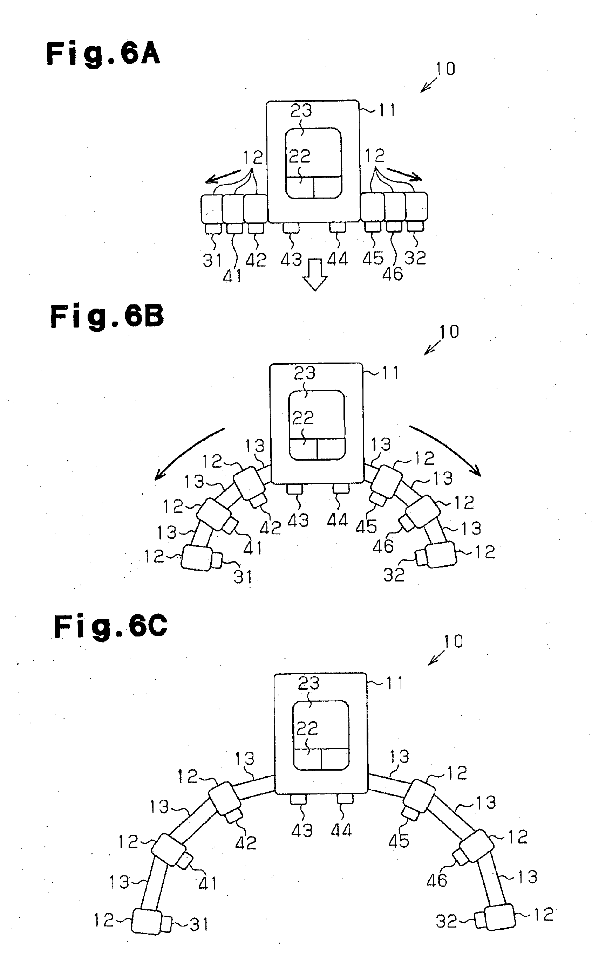

Referring to Figs. 5 and 6, preparation procedures prior to measurement will now be described.

Preparation Procedure 1: As illustrated in Fig. 5A, the body composition measurement device 1 is prepared. Here, the body fat measurement device 10 and the weight scale 80 are connected to each other.

Preparation Procedure 2: As illustrated in Fig. 5B, the body fat measurement device 10 is removed from the weight scale 80.

Preparation Procedure 3: As illustrated in Fig. 6A, the electrode seats 12 are drawn out from the two sides of the main body 11, and the distance between electrodes is lengthened.

Preparation Procedure 4: As illustrated in Fig. 6B, the connection portions 13 are extended in correspondence with the abdominal circumference and shape of the measured subject 60. More specifically, as illustrated in Fig. 7B, the connection portions 13 are extended so that the first current electrode 31 contacts the left flank 61 D of the measured subject 60 and the second current electrode 32 contacts the right flank 61C of the measured subject 60. In this state, the angles θ are adjusted so that the electrodes 31, 32, and 41 to 46 all contact the abdomen 61. Further, the extension amounts of the connection portions 13 are all equalized to equalize the distance between adjacent electrodes, that is, equalize the distance between the first current electrode 31 and the first voltage electrode 41, the distance between the first voltage electrode 41 and the second voltage electrode 42, the distance between the second voltage electrode 42 and the third voltage electrode 43, the distance between the fourth voltage electrode 44 and the fifth voltage electrode 45, the distance between the fifth voltage electrode 45 and the sixth voltage electrode 46, and the distance between the sixth voltage electrode 46 and the second current electrode 32.

Preparation Procedure 5: As illustrated in Fig. 6C, when the measured subject 60 has a large abdominal circumference, the connection portions 13 are further extended to increase the distance between electrodes.

With reference to Fig. 7, the measurement procedures will now be described.

Measurement Procedure 1: As illustrated in Fig. 7A, the measured subject 60 stands on the weight scale 80, while holding with his or her hands and pressing the body fat measurement device 10 against the abdomen 61. In this state, the electrodes 31, 32, and 41 to 46 are arranged in the horizontal direction.

Measurement Procedure 2: As illustrated in Fig. 7B, the measured subject 60 adjusts the position of the body fat measurement device 10 in the vertical direction so that the central part of the body fat measurement device 10 is aligned with the umbilicus 62 of the measured subject 60.

Measurement Procedure 3: The measured subject 60 operates the operation unit 22 to input various parameters including the abdominal circumference, age, and sex of the measured subject 60 and instructs the starting of the measurement.

When the measurement is started, the control unit 50 executes a control that applies current to the current electrode pair 30. As illustrated by arrow A in Fig. 7B, the current applied to the current electrode pair 30 flows across a deep location in the measured subject 60 between the left flank 61 D and the right flank 61C from the current electrode 31 to the current electrode 32. As the current flows through the measured subject 60, the voltage electrodes 41 to 46 detect voltages, at a number of locations, corresponding to the impedances of the composition of the measured subject 60, subcutaneous fat 71, visceral fat 72, and muscle 73. Here, the weight scale 80 measures the weight of the measured subject 60. An electrode layout in which a straight line connecting the first current electrode 31 and the second current electrode 32 extends across a relatively deep location in the measured subject 60 as illustrated in Fig. 7B may be referred to as an electrode layout that puts significance on visceral fat measurement.

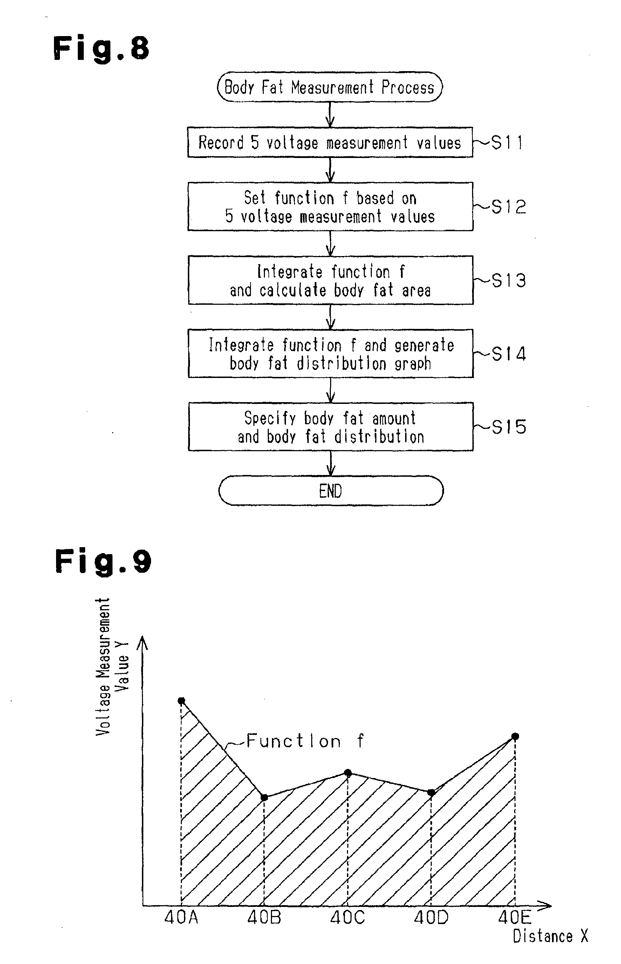

Referring to Fig. 8, the procedures of a body fat measurement process executed by the control unit 50 after measurement is started by the measuring person's instruction will now be described. This process is executed whenever measurement is started.

In step S11, when the current electrodes 31 and 32 supply the measured subject 60 with current, the control unit 50 stores voltage measurement values at five measurement points measured by the voltage electrode pair 40A, the voltage electrode pair 40B, the voltage electrode pair 40C, the voltage electrode pair 40D, and the voltage electrode pair 40E. In the preferred example, the control unit 50 stores each voltage measurement value in association with position information of the corresponding measurement point. For example, the position information of each measurement point may be the distance from a base point to a median position between the two voltage electrodes forming the corresponding one of the electrode pairs 40A to 40E. In a non-restrictive example, the base point is the position of the first current electrode 31.

In step S12, based on the five measurement values obtained in step S11, the control unit 50 sets a function f that connects the coordinates of the voltage measurement values as illustrated in Fig. 9. In Fig. 9, the vertical axis Y is a voltage measurement value, and the horizontal axis X is the distance from the current electrode 31 to each measurement point. In the illustrated example, the function f is set by performing linear interpolation on the coordinates of adjacent voltage measurement values.

More specifically, the distance from the current electrode 31, which is the base point, and the median position of the voltage electrode pair 40A is set as the X coordinate of the voltage electrode pair 40A. A value obtained by adding, to the X coordinate of the voltage electrode pair 40A, the distance from the median position of the voltage electrode pair 40A to the median position of the voltage electrode pair 40B is set as the X coordinate of the voltage electrode pair 40B. A value obtained by adding, to the X coordinate of the voltage electrode pair 40B, the distance from the median position of the voltage electrode pair 40B to the median position of the voltage electrode pair 40C is set as the X coordinate of the voltage electrode pair 40C. A value obtained by adding, to the X coordinate of the voltage electrode pair 40C, the distance from the median position of the voltage electrode pair 40C to the median position of the voltage electrode pair 40D is set as the X coordinate of the voltage electrode pair 40D. A value obtained by adding, to the X coordinate of the voltage electrode pair 40D, the distance from the median position of the voltage electrode pair 40D to the median position of the voltage electrode pair 40E is set as the X coordinate of the voltage electrode pair 40E. The control unit 50 calculates these distances and sets the coordinates.

The control unit 50 connects a plurality of linear functions that have different gradients and intercepts and connects the coordinates of the voltage measurement values corresponding to the electrode pair 40A, the electrode pair 40B, the electrode pair 40C, the electrode pair 40D, and the electrode pair 40E to set the function f. The distance between adjacent ones of the electrodes 40A to 40E may be default values that are set in advance. Alternatively, the control unit 50 may calculate the actual distance between electrodes from the measured value of the extension amount of each connection portion 13. The measured values of the extension amounts of the connection portions 13 correspond to the positions of the electrode pairs 40A to 40E relative to the measured subject 60. Thus, the function f may be set in conformance with the profile shape of the measured subject 60. The measured value of the extension amount of each connection portion 13 may be used to calculate the position of each measurement point.

In step S13, the control unit 50 integrates the function f set in step S12 to calculate the body fat area.

In step S14, the control unit 50 integrates the function f set in step S12 to generate a body fat distribution graph (e.g., part illustrated by diagonal lines in Fig. 9).

In step S50, the control unit 50 calculates the body fat amount and the body fat distribution based on the parameters of the measured subject input in measurement procedure 3 and an algorithm that is set in advance. Specifically, the control unit 50 specifies the body fat area calculated in step S13 and the body fat distribution graph generated in step S14 with an algorithm based on data collected in the past. As illustrated in Fig. 9, the measurement values of the voltage electrode pairs 40A and 40E arranged near the current electrodes 31 and 32 have a tendency of being higher relative to the amount of the actual body fat 70 than the measured values of the voltage electrode pairs 40B, 40C, and 40C arranged farther than the voltage electrode pairs 40A and 40E from the current electrodes 31 and 32. Thus, in step S15, the voltage measurement values can be corrected to values that are closer to the actual body fat. A graph obtained by correcting the graph of Fig. 9 can be used to check whether large amounts of the body fat 70 and the visceral fat 72 are distributed at the position of the electrode pair 40C, that is, in the vicinity of the umbilicus 62.

The graph illustrated in Fig. 9 as the calculation result of the body fat amount and body fat distribution calculated in step S15 is specified by an algorithm to generate a graph shown on the display unit 23.

As described above in detail, the body composition measurement device 1 of the first embodiment has the advantages described below.
(1) The control unit 50 sets the function f using the voltage measurement values of a plurality of measurement points and integrates the function f to measure the amount of the body fat 70. This allows for measurement of the body fat amount that reflects the distribution of the body fat 70 in the cross-section DA of the abdomen 61.
(2) The voltage electrode pairs 40A to 40E are arranged on the anterior belly 61 A. Thus, measurement values mainly reflecting the visceral fat 72 distributed in the anterior belly 61A can be obtained.
(3) The control unit 50 integrates the function f to calculate the area of the body fat 70. Thus, in contrast with the prior art example that performs a regression analysis on a single measurement value of a voltage electrode pair, the body composition measurement device 1 of the first embodiment can calculate the body fat amount that reflects the distribution of the body fat 70.
(4) The control unit 50 integrates the function f to estimate the body fat distribution and generates a distribution graph, which is the estimation result. Thus, the measuring person can be notified of the distribution of the body fat 70 on a cross-section of the abdomen 61.
(5) The body composition measurement device 1 measures the visceral fat 72 as the body fat 70. Thus, the amount and distribution of the visceral fat 72 can be shown to the measuring person in a diagram that reflects the distribution of the visceral fat 72 in the cross-section DA of the measured subject 60.
(6) The first current electrode 31 and the second current electrode 32, which form the current electrode pair 30, are arranged on opposite sides of the measured subject 60, that is, on the left flank 61 D and the right flank 61C. In this layout, the current applied to the current electrode pair 30 flows across a deep part of the measured subject 60. This allows for the voltage electrode pairs to obtain voltage measurement values mainly reflecting the visceral fat 72, a large amount of which is distributed at the center of the abdomen 61.
(7) The voltage electrode pairs 40A to 40E are arranged between the current electrode pair 30. This layout raises the voltage measurement sensitivity compared with when the voltage electrode pairs 40A to 40E are arranged at locations separated from the current electrode pair 30.
(8) In the abdomen 61, there is a tendency of visceral fat 72 forming most at the height of the umbilicus 62. In the present embodiment, the electrodes 31, 32, and 41 to 46 are arranged on a cross-section DA that extends through the umbilicus 62. This allows for the detection of voltages reflecting the portion where the visceral fat 72 is most formed. Generally, the determination of obesity or metabolic syndrome uses the body fat amount at the cross-sectional DA that extends through the umbilicus 62. Accordingly, the measurement result obtained by the body composition measurement device 1 is compared with a reference value such as that described above and is effective for determining obesity or metabolic syndrome.
(9) The connection portions 13 allow for the relative positions between voltage electrode pairs 40A to 40E to be changed. Thus, the same body fat measurement device 10 can measure the body fat amount of measured subjects 60 having different abdominal circumferences.

### Second Embodiment

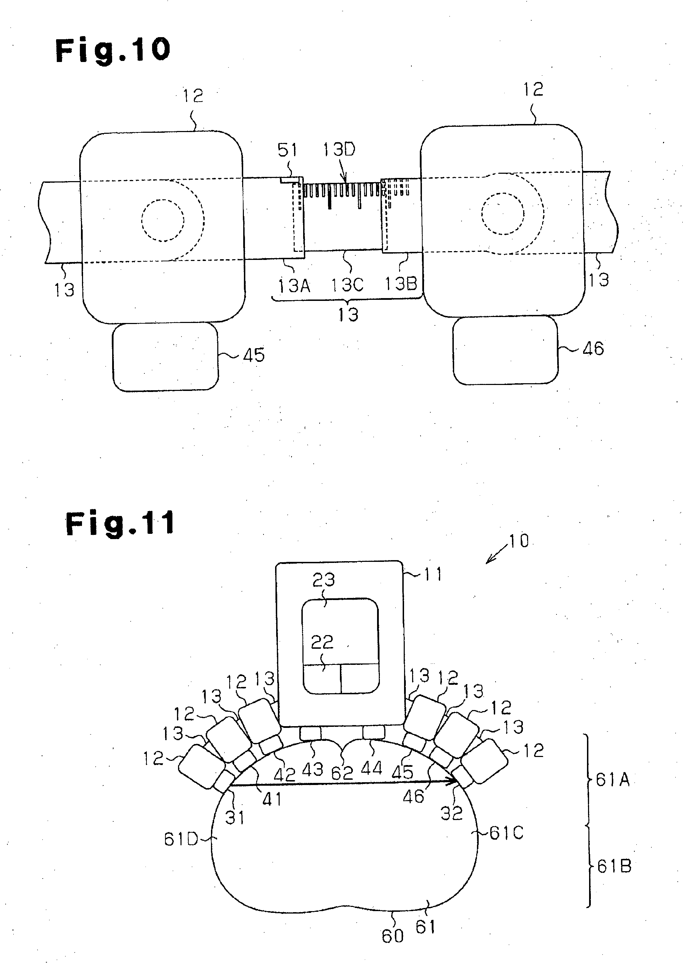

A second embodiment of the present invention will now be described with reference to Fig. 10.

In the second embodiment, the connection portion 13 of the first embodiment includes a distance sensor 51, which measures the extension amount of the connection portion 13. This different portion will now be described in detail. Otherwise, the structure is the same as the first embodiment, and the same reference numerals are given to those components that are the same as the corresponding components of the first embodiment. Such components will not be described.

In the example illustrated in Fig. 10, the distance sensor 51, which is arranged on the outer pipe 13A of each connection portion 13, includes a linear encoder that emits light toward the inner pipe 13B and the intermediate pipe 13C and receives the reflection light. The inner pipe 13B and the intermediate pipe 13C include a marking section 13D that includes marks formed at predetermined intervals.

When the inner pipe 13B moves relative to the outer pipe 13A, the distance sensor 51 provides the control unit 50 with detection values based on the reflection light that is periodically changed by the marks of the marking section 13D. The control unit 50 measures the movement amounts of the inner pipe 13B and the intermediate pipe 13C relative to the outer pipe 13A based on the detection values and calculates the positions of the inner pipe 13B and the intermediate pipe 13C relative to the outer pipe 13A, that is, calculates the extension amount of the connection portion 13.

As described above in detail, in addition to advantage (1) of the first embodiment, in which the body fat amount can be measured in correspondence with the distribution of the body fat 70 in a cross-section of the abdomen 61, and advantages (2) to (9), the second embodiment has the advantages described below.

(10) The control unit 50 uses the distance sensor 51 to calculate the distance between adjacent electrodes, that is, the extension amount of the connection portion. Thus, the measuring person is not required to visually check the extension amount of the connection portion 13.

(11) The control unit 50 calculates the abdominal circumference of the measured subject 60 based on the extension amount of each connection portion 13. Thus, in measurement procedure 3, instead of the abdominal circumference input by the measured subject 60, the calculation result of the abdominal circumference calculated by the control unit 50 can be used. This eliminates the need for the measured subject 60 to input the abdominal circumference.

### Third Embodiment

A third embodiment of the present invention will now be described with reference to Fig. 4.

In the third embodiment, the electrode seat 12 of the first embodiment accommodates an angle sensor 52 that measures the angle θ. The angle sensor 52 is one example of a distance measurement mechanism. This different portion will now be described in detail. Otherwise, the structure is the same as the first embodiment, and the same reference numerals are given to those components that are the same as the corresponding components of the first embodiment. Such components will not be described.

As illustrated in Fig. 4, the angle sensor 52 can be arranged on a connecting part of the connection portion 13 and may be a rotary potentiometer that outputs a voltage signal corresponding to the angle θ to the control unit 50. The control unit 50 calculates the angle θ based on the voltage signal from the angle sensor 52. The control unit 50 calculates the abdominal circumference of the measured subject 60 based on the angle θ calculated by the control unit 50 and an abdominal circumference map set in advance through experiments or the like. The angle θ increases as the abdominal circumference of the measured subject 60 increases. Thus, the abdominal calculation map is set in the control unit 50 as a map in which the abdominal circumference increases as the angle θ increases.

As described above in detail, in addition to advantage (1) of the first embodiment, in which the body fat amount can be measured in correspondence with the distribution of the body fat 70 in a cross-section of the abdomen 61, and advantages (2) to (9), the third embodiment has the advantages described below.

(12) The control unit 50 calculates the extension amount of the connection portion 13 based on the angle θ measured by the angle sensor 52. Thus, there is no need for the measuring person to visually check the extension amount of the connection portion 13.

(13) The control unit 50 calculates the abdominal circumference of the measured subject 60 based on the angle θ. Thus, in measurement procedure 3, instead of the abdominal circumference input by the measured subject 60, the abdominal circumference calculated based on the angle θ can be used. This eliminates the need for the measured subject 60 to input the abdominal circumference.

### Fourth Embodiment

A fourth embodiment of the present invention will now be described with reference to Fig. 11

In the fourth embodiment, the location of the electrodes relative to the measured subject 60 differs from the first embodiment. This different portion will now be described in detail. Otherwise, the structure is the same as the first embodiment, and the same reference numerals are given to those components that are the same as the corresponding components of the first embodiment. Such components will not be described.

As illustrated in Fig. 11, the first current electrode 31 is arranged at a position between the left flank 61 D and the umbilicus 62. The second current electrode 32 is arranged at a position between the right flank 61C and the umbilicus 62. The voltage electrodes 41 to 46 are sequentially arranged between the current electrode pair 30. Here, the inter-electrode distance between the first current electrode 31 and first voltage electrode 41, the inter-electrode distance between the first voltage electrode 41 and the second voltage electrode 42, the inter-electrode distance between the second voltage electrode 42 and the third voltage electrode 43, the inter-electrode distance between the fourth voltage electrode 44 and the fifth voltage electrode 45, the inter-electrode distance between the fifth voltage electrode 45 and the sixth voltage electrode 46, and the inter-electrode distance between the sixth voltage electrode 46 and the second current electrode 32 are equal.

During measurement, the current applied to the current electrode pair 30 flows across a deep part in the measured subject 60 and flows through a shallow part of the anterior belly 61A. Large amounts of the subcutaneous fat 71 are distributed at a location that is relatively shallow from the body surface (refer to Fig. 17). Accordingly, the voltage measurement values of the voltage electrode pairs 40A to 40E in the electrode layout of Fig. 11 mainly reflect the distribution of the subcutaneous fat 71. The electrode layout in which a straight line connecting the first current electrode 31 and the second current electrode 32 extends across a relatively shallow location in the measured subject 60 as illustrated in Fig. 11 may be referred to as an electrode layout that puts significance on subcutaneous fat measurement.

As described above in detail, in addition to advantage (1) of the first embodiment, in which the body fat amount can be measured in correspondence with the distribution of the body fat 70 in a cross-section of the abdomen 61, and advantages (3), (4), (7), and (9), the fourth embodiment has the advantages described below.

(14) The body composition measurement device 1 measures the subcutaneous fat 71 as the body fat 70. This allows for the fat amount and distribution diagram that reflects the distribution of the subcutaneous fat 71 in a measured cross-section of the measured subject 60 to be shown to the measuring person.

(15) The body composition measurement device 1 can measure the voltage that mainly reflects the amount of the subcutaneous fat 71, a large amount of which is distributed in a shallow portion of the measured subject 60, by decreasing the distance of the current electrode pair 30.

(16) The body composition measurement device 1 can arrange the electrodes 31, 32, and 41 to 46 in an electrode layout that puts significance on the subcutaneous fat measurement to obtain measurement results mainly reflecting the subcutaneous fat 71. Further, the body composition measurement device 1 can arrange the electrodes 31, 32, and 41 to 46 in an electrode layout that puts significance on the visceral fat measurement to obtain measurement results mainly reflecting the visceral fat 72.

The present invention is not limited to the foregoing embodiments, and the embodiments may be combined or modified as described below. Further, modified examples may also be combined.

The control unit 50 performs integration with the measurement values of the voltage electrode pair 40A to 40E to calculate the fat amount of the visceral fat 72 as the body fat 70. However, the fat amount and distribution of the body fat 70 obtained in the first embodiment may include the amount and distribution of the subcutaneous fat 71. Accordingly, the fat amount and distribution of the visceral fat 72 can be calculated reflecting the fat amount and distribution of the subcutaneous fat 71 measured by a subcutaneous fat measuring means that differs from the means used to measure the visceral amount. More specifically, in step S15 of Fig. 8, when the control unit 50 specifies the body fat amount and body fat distribution, the control unit 50 divides the fat amount and distribution of the subcutaneous fat 71 measured by the different subcutaneous fat measuring means by the body fat amount including the subcutaneous fat amount and visceral fat amount to correct the fat amount and distribution of the visceral fat 72.

As different subcutaneous fat measurement means, a method for having current flow to a shallow location of the measured subject with an electrode layout that puts significance on subcutaneous fat measurement as in the fourth embodiment, a method for measuring the subcutaneous fat 71 using near-infrared light, a method for estimating the subcutaneous fat 71 from the abdominal circumference of the measured subject 60, or the like may be employed. These different subcutaneous fat measuring means are implemented by the body fat measurement device 10, and the control unit 50 stores the measurement results of the different subcutaneous fat measuring means to correct the fat amount and distribution of the visceral fat 72. Further, the fat amount and distribution of the visceral fat 72 can be corrected by inputting the subcutaneous fat amount measured by a device that differs from the body fat measurement device 10 to the control unit 50 to correct the fat amount and distribution of the visceral fat 72.

In the second embodiment, an optical linear encoder is used as the distance sensor 51. However, a different sensor may be used. For example, the sensors described below in paragraphs (A) to (D) may be used.
(A) An ultrasonic distance meter that obtains the distance from one electrode seat 12 to the adjacent electrode seat 12 from the time required for ultrasonic waves to bounce back.
(B) A linear potentiometer that calculates distance from a voltage value that varies in correspondence with the extension amount of the connection portion 13.
(C) An optical distance meter that emits light waves from one electrode seat 12 to a reflection prism arranged on the adjacent electrode seat 12 and detects distance from the number of oscillations that occurs until the reflection light from the reflection prism is detected.
(D) A capacitance displacement meter that detects distance by converting capacitance, which is generated between a probe arranged on one electrode seat 12 to the adjacent electrode seat 12, into voltage.

In the second embodiment, the distance sensor 51 is arranged on each connection portion 13. However, the distance sensor 51 may be arranged on only one connection portion 13, and the abdominal circumference may be calculated based on the detection value of the distance sensor 51.

In the third embodiment, a rotary potentiometer is used as the angle sensor 52. However, a different sensor may be used. For example, a rotary encoder that detects a rotation angle by counting pulses output in correspondence with the rotational movement amount of one connection portion 13 relative to another connection portion 13 may be used.

In the third embodiment, the angle sensor 52 is arranged on each connection portion 13. However, the angle sensor 52 may be arranged on only one connection portion 13, and the abdominal circumference may be calculated based on the detection value of the angle sensor 52.

In the second embodiment, the distance sensor 51 is used to calculate the distance between adjacent electrodes. In the third embodiment, the angle sensor 52 is used to calculate the distance between adjacent electrodes. However, the distance between adjacent electrodes may be obtained in the following manner. Specifically, as a distance measurement mechanism, the connection portion 13 may include a mark corresponding to the extension amount of the connection portion, and the measuring person inputs the value of the mark to the operation unit 22.

The electrodes 31, 32, and 41 to 46 are arranged on the anterior belly 61A. However, at least one electrode maybe arranged at the side of the back 61B.

The control unit 50 performs linear interpolation on the coordinates of the measurement values of the voltage electrode pairs 40A to 40E to set the function f. However, the control unit 50 may set a function f that corresponds to an approximate curve based on the coordinates of the measurement values of the voltage electrode pairs 40A to 40E.

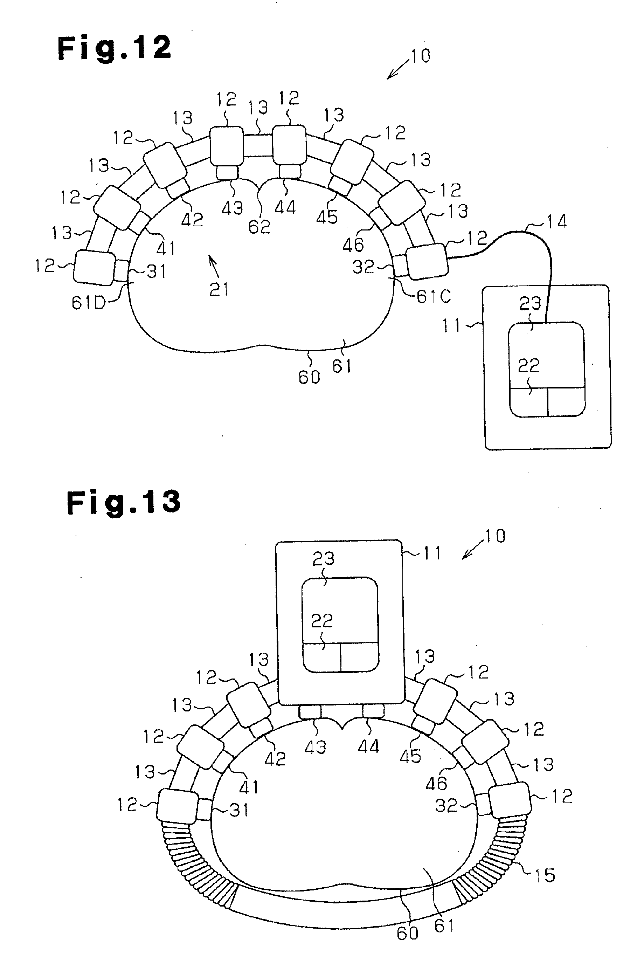

In each of the above embodiments, the electrode seat 12, which includes the detection unit 21, is connected to the main body 11. However, the electrode seat 12 may be discrete from the main body 11. Specifically, as illustrated in Fig. 12, the detection unit 21 includes the eight electrode seats 12, which correspond to the electrodes 31, 32, and 41 to 46, and the seven connection portions 13, which connect the adjacent electrode seats 12. The detection unit 21 and the main body 11 are connected by a cord 14 to form the body fat measurement device 10.

In each of the above embodiments, the body fat measurement device 10 includes the control unit 50 and the measurement portion 20. However, the weight scale 80 may include at least one of the control unit 50 and the measurement portion 20.

Each of the above embodiments employs the body composition measurement device 1 that combines the body fat measurement device 10 and the weight scale 80. However, the weight scale 80 may be eliminated.

In each of the above embodiments, the measured subject 60 holds the body fat measurement device 10 with his or her hands to perform a measurement. However, a belt may be arranged on the body fat measurement device 10 and fixed to the measured subject 60 to perform a measurement. More specifically, as illustrated in Fig. 13, the electrode seat 12 corresponding to the first current electrode 31 and the electrode seat 12 corresponding to the second current electrode 32 are connected by a belt 15 having an adjustable length. During measurement, the electrode seats 12, the connection portions 13, and the belt 15 fix the body fat measurement device 10 to the measured subject 60.

In each of the above embodiments, a connection portion 13 is not arranged between the third voltage electrode 43 and the fourth voltage electrode 44. However, a connection portion 13 may be arranged between the third voltage electrode 43 and the fourth voltage electrode 44, and the distance between the electrodes 43 and 44 may be varied.

In each of the above embodiment, the connection portions 13 connect the electrode seats 12. However, the connection portions 13 may be eliminated, and the electrodes may be independently arranged on the measured subject 60.

In each of the above embodiments, the connection portions 13, which can vary the distance between electrodes, connects the electrode seats 12. However, connection portions that cannot vary the distance between electrodes may be used to connect the electrode seats 12.

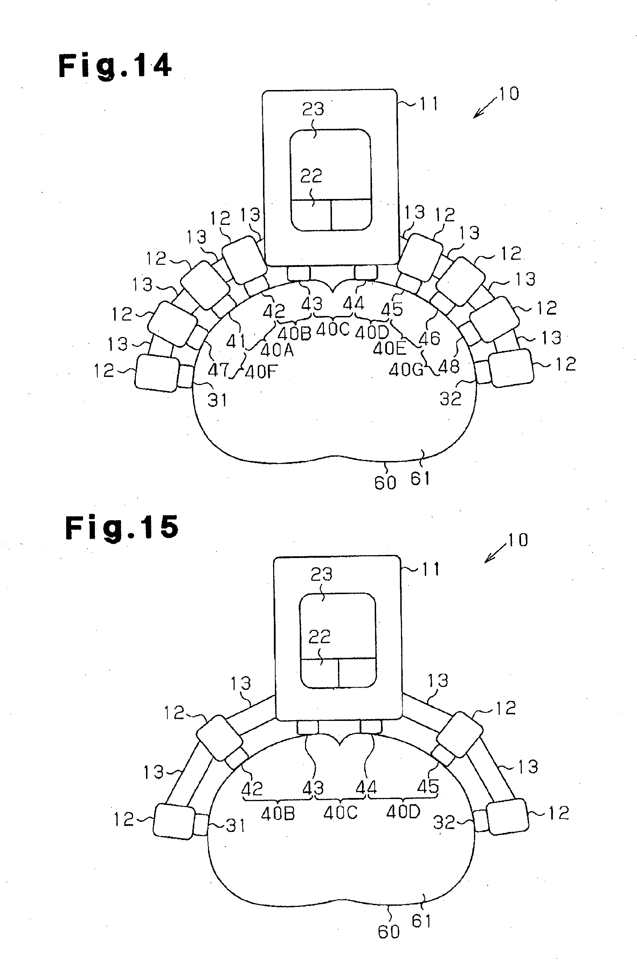

Each of the above embodiments includes the six voltage electrodes 41 to 46 but may include seven or more voltage electrodes. For example, as illustrated in Fig. 14, a seventh voltage electrode 47 and eighth voltage electrode 48 are arranged in addition to the voltage electrodes 41 to 46. The seventh voltage electrode 47 is arranged between the first current electrode 31 and the first voltage electrode 41. The eighth voltage electrode 48 is arranged between the sixth voltage electrode 46 and the second current electrode 32. Here, the function f is set based on the measurement values of a voltage electrode pair 40F, which includes the first voltage electrode 41 and the seventh voltage electrode 47, and the voltage electrode pair 40G, which includes the sixth voltage electrode 46 and the eighth voltage electrode 48, in addition to the measurement values of the voltage electrode pairs 40A to 40E.

Each of the above embodiments includes six voltage electrodes 41 to 46. However, one to three of the voltage electrodes may be eliminated. For example, as illustrated in Fig. 15, the first voltage electrode 41 and the sixth voltage electrode 46 are eliminated. Here, the control unit 50 sets the function f based on the measurement values of the voltage electrode pairs 40B, 40C, and 40D.

Each of the above embodiments includes the six voltage electrodes 41 to 46 that form the five voltage electrode pairs 40A to 40E. However, there may be only one voltage electrode pair that is movable relative to the current electrode pair 30 and the measured subject 60. In this case, the voltage electrode pair is moved to a number of locations to measure the voltage and the measurement values are sequentially stored. The control unit 50 sets the function f based on the measurement values.

In each of the above embodiments, the inter-electrode distances are equal between the first current electrode 31 and first voltage electrode 41, the first voltage electrode 41 and the second voltage electrode 42, the second voltage electrode 42 and the third voltage electrode 43, the fourth voltage electrode 44 and the fifth voltage electrode 45, the fifth voltage electrode 45 and the sixth voltage electrode 46, and the sixth voltage electrode 46 and the second current electrode 32. However, the inter-electrode distances may differ from one another.

In each of the above embodiments, the electrodes 31, 32, and 41 to 46 are arranged about the umbilicus 62, and the body fat amount is measured about the umbilicus 62. However, the electrodes 31, 32, and 41 to 46 may be arranged on only the right body half or the left body half. This allows for the body fat amount to be measured for only the right body half or the left body half. Further, in this state, based on the measurement result of one of the body halves obtained with the electrode pairs arranged on the body half, the body fat amount of the other body half may be estimated. Additionally, the entire body fat amount may be estimated based on the measurement amount of one body half. Moreover, by measuring each of the right body half and the left body half, the balance of the body fat amount between the right body half and the left body half can be checked.

In each of the above embodiments, the electrodes 31, 32, and 41 to 46 are arranged on the cross-sectional DA that extends through the umbilicus 62. However, the electrodes 31, 32, and 41 to 46 may be arranged on a different cross-section. More specifically, the electrodes 31, 32, and 41 to 46 may be arranged on a cross-section located on a cross-section located above or below the umbilicus 62. Further, electrodes 31, 32, and 41 to 46 may be arranged on cross-sections other than the abdomen 61, for example, any part other of the trunk other than the abdomen such as the thorax and any part of the trunk such as the thigh. Further, the electrodes 31, 32, and 41 to 46 may be arranged on a cross-section orthogonal or diagonal to the horizontal direction.

In each of the above embodiments, based on the measurement values of the voltage electrode pairs 40A to 40E arranged on the same cross-section, the body fat area and distribution of the body fat 70 on the same cross-section is calculated. However, the factors described below may be added to calculate the volume of the body fat as the body fat amount.

Specifically, the body fat measurement device 10 is moved along the vertical direction, and a function g is set based on a plurality of voltage measurement values taken on a cross-section DB, which differs from the cross-section DA that extends through the umbilicus 62 and is parallel to the cross-section DA. Then, the function g is integrated. Next, a function h is set in correspondence with a three-dimensional body obtained by connecting, on a three-dimensional graph, the parameters of the integration of the function f and the integration of the function g added to the distance X between electrodes and the voltage measurement value Y and the distance Z between the cross-sections. In other words, the function h is set with the distribution graph of the body fat 70 on the cross-section DA and the distribution graph of the body fat 70 on the cross-section DB. The function h is integrated in a range from the cross-sectional DA to the cross-section DB to calculate a three-dimensional distribution of the volume value of the body fat 70 that reflects the distribution of the body fat 70 from the cross-section DA to the cross-section DB.

Further, as a method for calculating the volume of the body fat 70, the above-described function h may be set, and the following calculation method may be implemented. One half of the distance between the cross-section DA and the cross-section DB is set as a distance L, and the volume of the body fat 70 is calculated from the sum of the product of the body fat area of the cross-section DA and distance L and the product of the body fat area of the cross-section DC and distance L.

In each of the above embodiments, the fat amount of the body fat 70 is shown as the measurement result of the body fat 70 as a graph on the display unit. However, the measurement result of the body fat 70 may be shown by numerals. As one example of such a case, the fat amount of the body fat 70 for each part of the measured subject 60 may be shown like "right abdomen: 50 cm³". Further, a cross-sectional diagram of the abdomen 61 may be shown, and the fat amount of the body fat 70 may be shown using colors, contrasts, and the like.

In each of the above embodiments, the measurement result of the visceral fat 72 is shown on the display unit 23. However, the method for conveying the measurement result to the measuring person is not limited in such a manner. For example, in addition to or instead of the display unit 23, a voice unit may be used to convey the measurement result to the measuring person with a voice.

In each of the above embodiments, telescopic members are used as the connection portions 13 so that the distance between the electrodes is variable. However, members allowing for sliding of the electrode seats and the connection portions relative to each other may be used so that the distance between the electrodes is variable.

In each of the above embodiments, the electrode material is not limited to a stainless steel alloy or a metal-plated resin and may be a gel.

In each of the above embodiments, the measurement portion 20 incorporates a power supply. However, the body fat measurement device 10 may be supplied with power from an outer power supply.

In each of the above embodiments, a measurement is performed in a state in which the measured subject 60 is standing. However, the visceral fat 72 may be measured in accordance with the measurement method of each of the above embodiments when the measured subject 60 is in a sitting or supine state.

The measured subject 60 is not limited to a human body and may be an animal

## Claims

1. A body composition measurement device comprising:
a current electrode pair (30) configured to apply current;
a plurality of voltage electrode pairs (40A-40E) configured to measure voltages at a plurality of measurement points; and a control unit (50) configured to perform an integration using measurement values of the measurement points measured by the voltage electrode pairs (40A-40E) and calculate a body fat amount based on the result of the integration; and
**characterized by**
a plurality of electrode seats (12) respectively connected to electrodes (31, 32, 41-46) forming the current electrode pair (30) and the plurality of voltage electrode pairs (40A-40E);
a plurality of connection portions (13) each including a variable length structure (13A, 13B, 13C) formed by a set of interconnecting pipes each movable in the axial direction and each said connection portion connecting two adjacent ones of the plurality of electrode seats (12) so that the plurality of electrode seats (12) are arranged in series.

2. The body composition measurement device according to claim 1, wherein the voltage electrode pairs (40A-40E) are configured to measure voltages for a measured subject in the vicinity of a frontal plane and at an anterior belly.

3. The body composition measurement device according to claim 1 or 2, wherein the control unit (50) is configured to calculate, as the body fat amount, an area of body fat in a measured cross-section of a measured subject.

4. The body composition measurement device according to any one of claims 1 to 3, wherein the control unit (50) is configured to estimate a distribution of body fat in a measured cross-section of a measured subject.

5. The body composition measurement device according to any one of claims 1 to 4, wherein the control unit (50) is configured to measure visceral fat amount as the body fat amount.

6. The body composition measurement device according to any one of claims 1 to 5, wherein the control unit (50) is configured to measure subcutaneous fat amount as the body fat amount.

7. The body composition measurement device according to any one of claims 1 to 6, wherein
the control unit (50) is configured to perform a first integration using measurement values of a plurality of measurement points on a first measured cross-section of a measured subject and a second integration using measurement values of a plurality of measurement points on a second measured cross-section, and
the control unit (50) is configured to calculate a body fat volume as the body fat amount based on a calculation result of the first integration and a calculation result of the second integration.

8. The body composition measurement device according to any one of claims 1 to 7, wherein
the current electrode pair (30) includes a first current electrode (31) and a second current electrode (32) arranged on opposite sides of a measured subject, and
the voltage electrode pairs (40A-40E) are arranged between the first current electrode (31) and the second current electrode (32).

9. The body composition measurement device according to any one of claims 1 to 8, wherein the current electrode pair (30) includes a first current electrode (31), which is arranged on a frontal plane of a right body half of a measured subject, and a second current electrode (32), which is arranged on a frontal plane of a left body half.

10. The body composition measurement device according to any one of claim 5 and claims 6 to 9, which depend on claim 5, comprising a subcutaneous fat measurement means configured to measure subcutaneous fat, wherein the control unit (50) is configured to calculate the visceral fat amount as the body fat amount that reflects the subcutaneous fat amount.

11. The body composition measurement device according to any one of claims 1 to 10, wherein
a distance is variable between two voltage electrodes of at least one of the voltage electrode pairs, and
the control unit (50) is configured to reflect the distance in the integration.

12. The body composition measurement device according to any one of claims 1 to 11, further comprising a distance measurement mechanism (51, 52) configured to measure the distance between the current electrode pair (30) and two adjacent ones of electrodes forming the voltage electrode pairs (40A-40E).

13. The body composition measurement device according to claim 12, wherein the distance measurement mechanism (51, 52) includes a distance sensor configured to measure the distance between the two adjacent ones of electrodes.

14. The body composition measurement device according to claim 12, wherein the plurality of connection portions (13) includes:
a first connection portion that connects a first voltage electrode and a second voltage electrode, and
a second connection portion that connects the first voltage electrode and a third voltage electrode,
wherein the distance measurement mechanism (51, 52) includes an angle sensor (52) configured to detect an angle between the first connection portion and the second connection portion.

15. The body composition measurement device according to any one of claims 12 to 14, wherein the control unit (50) is configured to estimate an abdominal circumference based on the distance between the two adjacent ones of the electrodes measured by the distance measurement mechanism.

16. The body composition measurement device according to claim 1, wherein the control unit (50) is configured to store the measurement values and positions of the measurement points in association with the corresponding measurement values and calculate the body fat amount based on the positions of the measurement points and the measurement values corresponding to the positions of the measurement points.

17. The body composition measurement device according to claim 1, wherein
the control unit (50) is connected to the current electrode pair and the voltage electrode pairs,
the plurality of electrodes forming the current electrode pair (30) and the voltage electrode pairs (40A-40E) are arranged in series and can be arranged on an arc forming part of a profile of a measured cross-section of the measured subject, and
the control unit (50) is configured to perform an integration based on measurement values measured by the voltage electrode pairs and positions of the measurement points to calculate a body fat amount.

## Patentansprüche

1. Vorrichtung zum Messen einer Körperzusammensetzung, die umfasst:
ein Strom-Elektrodenpaar (30), das so eingerichtet ist, dass es Strom anlegt;
eine Vielzahl von Spannungs-Elektrodenpaaren (40A-40E), die so eingerichtet sind, dass sie Spannungen an einer Vielzahl von Messpunkten messen; und
eine Steuereinheit (50), die so eingerichtet ist, dass sie eine Integration unter Verwendung durch die Spannungs-Elektrodenpaare (40A-40E) gemessener Messwerte der Messpunkte durchführt und eine Menge des Körperfetts auf Basis des Ergebnisses der Integration berechnet; und
**gekennzeichnet durch**
eine Vielzahl von Elektroden-Aufnahmen (12), die jeweils mit Elektroden (31, 32, 41-46) verbunden sind, die das Strom-Elektrodenpaar (30) und die Vielzahl von Spannungs-Elektrodenpaaren (40A-40E) bilden;
eine Vielzahl von Verbindungsabschnitten (13), die jeweils eine Struktur (13A, 13B, 13C) veränderlicher Länge enthalten, die **durch** eine Gruppe von Verbindungsrohren gebildet wird, die jeweils in der axialen Richtung bewegt werden können, wobei jeder der Verbindungsabschnitte zwei benachbarte der Vielzahl von Elektrodenaufnahmen (12) so verbindet, dass die Vielzahl von Elektrodenaufnahmen (12) in Reihe angeordnet sind.

2. Vorrichtung zum Messen einer Körperzusammensetzung nach Anspruch 1, wobei die Spannungs-Elektrodenpaare (40A-40E) so eingerichtet sind, dass sie Spannungen für eine zu messende Person in der Nähe einer Frontalebene und an einem vorderen Bauch (anterior belly) messen.

3. Vorrichtung zum Messen einer Körperzusammensetzung nach Anspruch 1 oder 2, wobei die Steuereinheit (50) so eingerichtet ist, dass sie als die Menge des Körperfetts eine Fläche von Körperfett in einem gemessenen Querschnitt einer gemessenen Person berechnet.

4. Vorrichtung zum Messen einer Körperzusammensetzung nach einem der Ansprüche 1 bis 3, wobei die Steuereinheit (50) so eingerichtet ist, dass sie eine Verteilung von Körperfett in einem gemessenen Querschnitt einer gemessenen Person schätzt.

5. Vorrichtung zum Messen einer Körperzusammensetzung nach einem der Ansprüche 1 bis 4, wobei die Steuereinheit (50) so eingerichtet ist, dass sie eine Menge des Bauchfetts als die Menge des Körperfetts misst.

6. Vorrichtung zum Messen einer Körperzusammensetzung nach einem der Ansprüche 1 bis 5, wobei die Steuereinheit (50) so eingerichtet ist, dass sie eine Menge des Unterhautfetts als die Menge des Körperfetts misst.

7. Vorrichtung zum Messen einer Körperzusammensetzung nach einem der Ansprüche 1 bis 6, wobei
die Steuereinheit (50) so eingerichtet ist, dass sie eine erste Integration unter Verwendung von Messwerten einer Vielzahl von Messpunkten an einem ersten gemessenen Querschnitt einer gemessenen Person und eine zweite Integration unter Verwendung von Messwerten einer Vielzahl von Messpunkten an einem zweiten gemessenen Querschnitt durchführt, und
die Steuereinheit (50) so eingerichtet ist, dass sie ein Volumen des Körperfetts als die Menge des Körperfetts auf Basis eines Berechnungsergebnisses der ersten Integration und eines Berechnungsergebnisses der zweiten Integration berechnet.

8. Vorrichtung zum Messen einer Körperzusammensetzung nach einem der Ansprüche 1 bis 7, wobei
das Strom-Elektrodenpaar (30) eine erste Strom-Elektrode (31) und eine zweite Strom-Elektrode (32) enthält, die an einander gegenüberliegenden Seiten einer gemessenen Person angeordnet sind, und
die Spannungs-Elektrodenpaare (40A-40E) zwischen der ersten Strom-Elektrode (31) und der zweiten Strom-Elektrode (32) angeordnet sind.

9. Vorrichtung zum Messen einer Körperzusammensetzung nach einem der Ansprüche 1 bis 8, wobei das Strom-Elektrodenpaar (30) eine erste Strom-Elektrode (31), die an einer Frontalebene einer rechten Körperhälfte einer gemessenen Person angeordnet ist, und eine zweite Strom-Elektrode (32) enthält, die an einer Frontalebene einer linken Körperhälfte angeordnet ist.

10. Vorrichtung zum Messen einer Körperzusammensetzung nach Anspruch 5 und den Ansprüchen 6 bis 9, die von Anspruch 5 abhängig sind, die eine Einrichtung zum Messen von Unterhautfett umfasst, die so eingerichtet ist, dass sie Unterhautfett misst, wobei die Steuereinheit (50) so eingerichtet ist, dass sie die Menge des Bauchfetts als die Menge des Körperfetts berechnet, die die Menge des Unterhautfetts widerspiegelt.

11. Vorrichtung zum Messen einer Körperzusammensetzung nach einem der Ansprüche 1 bis 10, wobei
ein Abstand zwischen zwei Spannungs-Elektroden wenigstens eines der Spannungs-Elektrodenpaare verändert werden kann, und
die Steuereinheit (50) so eingerichtet ist, dass sie den Abstand in der Integration widerspiegelt.

12. Vorrichtung zum Messen einer Körperzusammensetzung nach einem der Ansprüche 1 bis 11, die des Weiteren einen Mechanismus (51, 52) zum Messen eines Abstandes umfasst, der so eingerichtet ist, dass er den Abstand zwischen dem Strom-Elektrodenpaar (30) und zwei benachbarten Elektroden misst, die die Spannungs-Elektrodenpaare (40A-40E) bilden.

13. Vorrichtung zum Messen einer Körperzusammensetzung nach Anspruch 12, wobei der Mechanismus (51, 52) zum Messen eines Abstandes einen Abstandssensor enthält, der so eingerichtet ist, dass er den Abstand zwischen den zwei benachbarten Elektroden misst.

14. Vorrichtung zum Messen einer Körperzusammensetzung nach Anspruch 12, wobei die Vielzahl von Verbindungsabschnitten (13) enthält:
einen ersten Verbindungsabschnitt, der eine erste Spannungs-Elektrode und eine zweite Spannungs-Elektrode verbindet, und
einen zweiten Verbindungsabschnitt, der die erste Spannungs-Elektrode und eine dritte Spannungs-Elektrode verbindet,
wobei der Mechanismus (51, 52) zum Messen eines Abstandes einen Winkelsensor (52) enthält, der so eingerichtet ist, dass er einen Winkel zwischen dem ersten Verbindungsabschnitt und dem zweiten Verbindungsabschnitt erfasst.

15. Vorrichtung zum Messen einer Körperzusammensetzung nach einem der Ansprüche 12 bis 14, wobei die Steuereinheit (50) so eingerichtet ist, dass sie einen Bauchumfang auf Basis des durch den Mechanismus zum Messen eines Abstandes gemessenen Abstandes zwischen den zwei benachbarten der Elektroden schätzt.

16. Vorrichtung zum Messen einer Körperzusammensetzung nach Anspruch 1, wobei die Steuereinheit (50) so eingerichtet ist, dass sie die Messwerte und Positionen der Messpunkte in Verbindung mit den entsprechenden Messwerten speichert und die Menge des Körperfetts auf Basis der Positionen der Messpunkte sowie der Messwerte berechnet, die den Positionen der Messpunkte entsprechen.

17. Vorrichtung zum Messen einer Körperzusammensetzung nach Anspruch 1, wobei
die Steuereinheit (50) mit dem Strom-Elektrodenpaar und den Spannungs-Elektrodenpaaren verbunden ist,
die Vielzahl von Elektroden, die das Strom-Elektrodenpaar (30) und die Spannungs-Elektrodenpaare (40A-40E) bilden, in Reihe angeordnet sind und auf einem Bogen angeordnet sein können, der Teil eines Profils eines gemessenen Querschnitts der gemessenen Person ist, und
die Steuereinheit (50) so eingerichtet ist, dass sie eine Integration auf Basis durch die Spannungs-Elektrodenpaare gemessener Messwerte und Positionen der Messpunkte durchführt, um eine Menge des Körperfetts zu berechnen.

## Revendications

1. Dispositif de mesure de la composition corporelle comprenant :
une paire d'électrodes de courant (30) configurées pour appliquer un courant ;
une pluralité de paires d'électrodes de tension (40A à 40E) configurées pour mesurer des tensions en une pluralité de points de mesure ; et
une unité de commande (50) configurée pour effectuer une intégration en utilisant les valeurs de mesure des points de mesure mesurées par les paires d'électrodes de tension (40A à 40E) et pour calculer la teneur en graisse corporelle en se basant sur le résultat de l'intégration ; et
**caractérisé par**
une pluralité de supports d'électrode (12) respectivement connectés à des électrodes (31, 32, 41 46) formant la paire d'électrodes de courant (30) et la pluralité de paires d'électrodes de tension (40A à 40E) ;
une pluralité de parties de connexion (13) incluant chacune une structure de longueur variable (13A, 13B, 13C) formée par un ensemble de tuyaux d'interconnexion, chacun étant mobile dans la direction axiale et chaque dite partie de connexion reliant deux supports adjacents de la pluralité de supports d'électrode (12), de sorte que les supports de la pluralité de supports d'électrode (12) sont agencés en série.

2. Dispositif de mesure de la composition corporelle selon la revendication 1, dans lequel les électrodes de la paire d'électrodes de tension (40A à 40E) sont configurées pour mesurer les tensions pour un patient, mesurées au voisinage du plan frontal et du ventre antérieur.

3. Dispositif de mesure de la composition corporelle selon la revendication 1 ou 2, dans lequel l'unité de commande (50) est configurée pour calculer, en tant que teneur en graisse corporelle, la surface de la graisse corporelle dans une section mesurée d'un patient mesuré.

4. Dispositif de mesure de la composition corporelle selon l'une quelconque des revendications 1 à 3, dans lequel l'unité de commande (50) est configurée pour estimer la répartition de la graisse corporelle dans une section mesurée d'un patient mesuré.

5. Dispositif de mesure de la composition corporelle selon l'une quelconque des revendications 1 à 4, dans lequel l'unité de commande (50) est configurée pour mesurer la teneur en graisse viscérale en tant que teneur en graisse corporelle.

6. Dispositif de mesure de la composition corporelle selon l'une quelconque des revendications 1 à 5, dans lequel l'unité de commande (50) est configurée pour mesurer la teneur en graisse sous-cutanée en tant que teneur en graisse corporelle.

7. Dispositif de mesure de la composition corporelle selon l'une quelconque des revendications 1 à 6, dans lequel
l'unité de commande (50) est configurée pour effectuer une première intégration en utilisant les valeurs de mesure d'une pluralité de points de mesure sur une première section mesurée d'un patient mesuré et une seconde intégration utilisant les valeurs de mesure d'une pluralité de points de mesure sur une seconde section mesurée, et
l'unité de commande (50) est configurée pour calculer le volume de graisse corporelle en tant que teneur en graisse corporelle en se basant sur le résultat de calcul de la première intégration et sur le résultat de calcul de la seconde intégration.

8. Dispositif de mesure de la composition corporelle selon l'une quelconque des revendications 1 à 7, dans lequel
la paire d'électrodes de courant (30) comporte une première électrode de courant (31) et une deuxième électrode de courant (32) agencées sur les flancs opposés d'un patient mesuré, et
les paires d'électrodes de tension (40A à 40E) sont agencées entre la première électrode de courant (31) et la deuxième électrode de courant (32).

9. Dispositif de mesure de la composition corporelle selon l'une quelconque des revendications 1 à 8, dans lequel la paire d'électrodes de courant (30) comporte une première électrode de courant (31) qui est agencée sur le plan frontal de la moitié droite du corps d'un patient mesuré et une deuxième électrode de courant (32) qui est agencée sur le plan frontal de la moitié gauche du corps.

10. Dispositif de mesure de la composition corporelle selon l'une quelconque de la revendication 5 et des revendications 6 à 9, dépendant de la revendication 5, comprenant un moyen de mesure de graisse sous-cutanée configuré pour mesurer la graisse sous-cutanée, dans lequel l'unité de commande (50) est configurée pour calculer la teneur en graisse viscérale en tant que teneur en graisse corporelle qui répercute la teneur en graisse sous-cutanée.

11. Dispositif de mesure de la composition corporelle selon l'une quelconque des revendications 1 à 10, dans lequel
la distance entre deux électrodes de tension d'au moins l'une des paires d'électrodes de tension est variable, et
l'unité de commande (50) est configurée pour répercuter la distance dans l'intégration.

12. Dispositif de mesure de la composition corporelle selon l'une quelconque des revendications 1 à 11, comprenant en outre un mécanisme de mesure de distance (51, 52) configuré pour mesurer la distance entre les électrodes de la paire d'électrodes de courant (30) et deux électrodes adjacentes des électrodes formant les paires d'électrodes de tension (40A à 40E).

13. Dispositif de mesure de la composition corporelle selon la revendication 12, dans lequel le mécanisme de mesure de distance (51, 52) comporte un capteur de distance configuré pour mesurer la distance entre les deux électrodes adjacentes.

14. Dispositif de mesure de la composition corporelle selon la revendication 12, dans lequel la pluralité de parties de connexion (13) comporte :
une première partie de connexion qui connecte une première électrode de tension et une deuxième électrode de tension, et
une seconde partie de connexion qui connecte la première électrode de tension et une troisième électrode de tension,
dans lequel le mécanisme de mesure de distance (51, 52) comporte un capteur angulaire (52) configuré pour détecter l'angle entre la première partie de connexion et la seconde partie de connexion.

15. Dispositif de mesure de la composition corporelle selon l'une quelconque des revendications 12 à 14, dans lequel l'unité de commande (50) est configurée pour estimer la circonférence abdominale en se basant sur la distance entre les deux électrodes adjacentes mesurée par le mécanisme de mesure de distance.

16. Dispositif de mesure de la composition corporelle selon la revendication 1, dans lequel l'unité de commande (50) est configurée pour mémoriser les valeurs de mesure et les positions des points de mesure en association avec les valeurs de mesure correspondantes et pour calculer la teneur en graisse corporelle en se basant sur les positions des points de mesure et les valeurs de mesure correspondant aux positions des points de mesure.

17. Dispositif de mesure de la composition corporelle selon la revendication 1, dans lequel
l'unité de commande (50) est connectée à la paire d'électrodes de courant et aux paires d'électrodes de tension,
les électrodes de la pluralité d'électrodes formant la paire d'électrodes de courant (30) et les paires d'électrodes de tension (40A à 40E) sont agencées en série et peuvent être agencées sur un arc faisant partie d'un profil de section mesuré du patient mesuré, et
l'unité de commande (50) est configurée pour effectuer une intégration en se basant sur les valeurs de mesure mesurées par les paires d'électrodes de tension et les positions des points de mesure pour calculer la teneur en graisse corporelle.
